(19) Europäisches Patentamt
European Patent Office
Office européen des brevets

(11) Publication number: **0 037 224**
**A1**

(12) # EUROPEAN PATENT APPLICATION

(21) Application number: 81301219.2

(22) Date of filing: 23.03.81

(51) Int. Cl.³: C 11 D 3/48
C 11 D 1/88, A 61 K 7/16
A 61 K 31/09, C 07 C 51/41
A 01 N 33/00, B 01 F 17/00

(30) Priority: 24.03.80 US 133380

(43) Date of publication of application: 07.10.81 Bulletin 81/40

(84) Designated Contracting States: BE DE FR GB IT NL

(71) Applicant: THE PROCTER & GAMBLE COMPANY
301 East Sixth Street
Cincinnati Ohio 45201(US)

(72) Inventor: Berg, Ronald Wayne
6353 Delcrest Drive
Fairfield, OH 45014(US)

(72) Inventor: Zimmerer, Roger Earl
8891 Woodview Drive
Cincinnati Ohio 45231(US)

(74) Representative: Brooks, Maxim Courtney et al,
Procter & Gamble (NTC) Limited Whitley Road
Longbenton
Newcastle-upon-Tyne NE12 9TS(GB)

(54) **Rare earth metal carboxylates, their use as antimicrobial agents, and medicinal, cosmetic and cleansing compositions containing them.**

(57) Compounds formed by combining rare earth metals with anionic or zwitterionic carboxylate surfactants are disclosed. The compounds herein provide broad spectrum antimicrobial benefits and are useful in a wide variety of medicinal, cosmetic and cleansing compositions.

EP 0 037 224 A1

# Rare Earth Metal Carboxylates, Their Use As Antimicrobial Agents, And Medicinal, Cosmetic And Cleansing Compositions Containing Them

The desirability of controlling or eradicating common disease-causing microorganisms on, e.g., the bodies of humans and lower animals, textiles, hard surfaces, etc., is clearly accepted. For use on humans and other animals, it is highly desirable to provide compositions for controlling microorganisms without harming the host. When the microorganisms are bacteria, it is usually desirable to use antimicrobial agents that are capable of killing or controlling both Gram-positive and Gram-negative species.

The present invention provides rare earth metal/carboxylate surfactant compounds which are excellent antimicrobial agents for use in a wide variety of products.

Various cationic and halogenated antimicrobial agents are known in the art. However, the present compounds offer several advantages over the art-disclosed antimicrobials. The compounds herein are less toxic, orally, than the common cationic surfactant-type antimicrobials, and they are less persistent in the environment than the halogenated antimicrobials. In contrast with many of the art-disclosed antimicrobials, microorganisms do not appear to form mutant strains resistant to the present compounds. The compounds herein can be used at low concentrations to control both Gram-positive and Gram-negative bacteria, and they are acceptably mild even when relatively high concentrations are used in contact with skin. Moreover, they kill or control bacteria with only minimal contact time. Accordingly, the antimicrobial compounds herein are especially useful in hard surface cleaners, cosmetics and oral compositions such as dentifrices and mouthwashes.

## Background Art

Belgian Patent 856,751 discloses cerium salts of sulfadiazine, especially cerous sulfadiazine, for treating burns. These salts are obtained from reacting water-soluble salts of cerium, for example, cerium chloride or cerium nitrate, with an aqueous solution of sulfadiazine, for example, sodium sulfadiazine. The cerium salt of sulfadiazine is said to display antibacterial activity at low concentrations of 0.05 micromole/ml.

A. Yu Zimakov, et al., RARE EARTH ELEMENTS IN MEDICINE (Kazanskii Meditsinskii Zhurnal, 57(3):268-271 (1976)), relates generally to the antibacterial activity of rare earth metals. More specifically, this article discusses the antibacterial activity of inorganic salts of rare earth metals, e.g., cerium nitrate at 1000 ppm inhibits Staphylococcus and molds. Reference was made to the therapeutic benefits of sulfates of neodymium, praseodymium, samarium and acetates of lanthanum in the treatment of tuberculosis.

Sudi Burkes, et al., THE BACTERIOSTATIC ACTIVITY OF CERIUM, LANTHANUM, AND THALLIUM, Journal of Bacteriology 54:417-24 (1947), reports the bacteriostatic properties of the captioned rare earth metals at concentrations ranging from 0.0006 to 0.0012 M. Inorganic salts of the captioned rare earth metals were found to have bacteriostatic properties at these concentrations.

Charles Levinson, et al., LANTHANUM-INDUCED ALTERATION IN CELLULAR ELECTROLYTES AND MEMBRANE POTENTIAL IN EHRLICH ASCITES TUMOR CELLS, Journal of Cellular Physiology 79:299-308 (1971), reports the effect of lanthanum at varying concentrations from 0.01 to 2.0 mM on membrane potential and electrolyte composition of Ehrlich ascites tumor cells. $La^{+3}$ concentrations less than 0.02 mM had no effect. At 1.0 mM the effect was maximal and resulted in an 87% reduction in cellular potassium, 79%

reduction in chloride ions and 21% reduction in sodium within 4.8 minutes. The sodium loss occurred even in the face of an electrochemical potential gradient favoring sodium entry. In short, this article shows that $La^{+3}$ affects the permeability of animal cell membranes.

Hunt, U.S. Patent 3,773,813, discloses a process for preparing anionic metal sulfonates. Lanthanum and yttrium are disclosed as some of the metal constituents. These anionic metal sulfonates are put to use as standards for spectographic equipment.

Conostan, a technical publication by the Conostan Division, (Continental Oil Company (Oklahoma)) discloses the inclusion of lanthanum anionic surfactant combinations in oil as internal standards for spectrometric analysis.

Belgian Patent 868,250, Imperial Chemical Industries Ltd., discloses that teeth are cleaned by applying the cations of one or more elements chosen from yttrium, scandium, lanthanum, cerium, praseodymium, neodymium, promethium, samarium, europium, gadolinium, terbium, dysprosium, holmium, erbium, lutetium, thulium, ytterbium. The patent discloses that the cation may be used in the form of a salt, which is preferably water soluble, or in the form of a salt with an anion that has an antibacterial effect. The preferred compound is said to be lanthanum chloride.

British Patent 1,475,971, Wella Aktiengesellschraft, teaches that cerium IV salts and their reaction products with anionic, cationic or amphoteric surfactants, can be used to remove reducing or oxidizing residues from hair to prevent damage to its structure or appearance. The compositions are also said to have disinfecting and deodorizing properties.

Jones, U.S. Patent 2,113,842, describes the use of a rare earth salt of a sulfonate of a higher alcohol to raise the surface tension in embalming fluids. A rate earth lauryl sulfonate is disclosed on page 2.

## Summary of the Invention

The present invention encompasses water-soluble compounds of the formula

$$(RCO_2^-)_3M^{3+} ,$$

wherein M is a rare earth metal cation and R is a $C_8$-$C_{18}$ hydrocarbyl ethoxylate group containing from about 1 to about 15 ethylene oxide units, a $C_8$-$C_{18}$ hydrocarbyl glyceryl ether group, a $C_8$-$C_{18}$ hydrocarbyl monoglyceride group, a $C_8$-$C_{18}$ hydrocarbyl ester group, or an ammonium phosphonium or sulfonium group of the formula

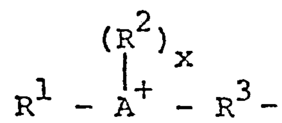
or
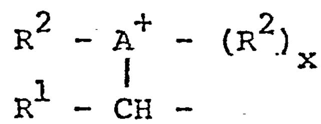
,

wherein $R^1$ is a $C_8$-$C_{22}$ hydrocarbyl group, each $R^2$ is a $C_1$-$C_4$ hydrocarbyl group or an ethylene oxide group containing from 1 to about 5 ethylene oxide units, x is 2 when A is N or P and x is 1 when A is S, and $R^3$ is a $C_1$-$C_{10}$ hydrocarbyl group or an ethylene oxide group containing from 1 to about 10 ethylene oxide units.

The invention also encompasses antimicrobial compositions comprising the above-described compounds.

The invention further encompasses methods for controlling microorganisms comprising contacting said microorganisms with a microorganism-controlling amount of the compounds or compositions of the foregoing type.

## Detailed Description of the Invention

The present invention is based on the discovery that compounds formed by combining certain rare earth metals and anionic or zwitterionic carboxylate surfactants possess synergistic antimicrobial properties. (The term "synergistic" as used herein means that combinations of the two discrete entities, i.e., the rate earth metal and the surfactant, provide antimicrobial activity far greater than either entity alone.)

Inorganic rare earth salts such as the lanthanum salts do not, by themselves, possess appreciable bactericidal activity. This is demonstrated by the following data obtained from standard tests for measuring Minimum Bactericidal Concentrations (using a 10-minute contact with the test agent at 37°C; representing at least a 3 log unit reduction in viable cell count).

Minimum Bactericidal Concentration

| Compound | E. coli | S. aureus |
|---|---|---|
| $LaCl_3.6H_2O$ | >3500 ppm | >3500 ppm |
| $La(NO_3)_3.6H_2O$ | >4300 ppm | >4300 ppm |
| $La_2(SO_4)_3.8H_2O$ | >7100 ppm | >7100 ppm |

Certain anionic and zwitterionic surfactants are somewhat bactericidal to Gram-positive bacteria at moderate to high concentrations, but none possess appreciable activity against Gram-negative bacteria. However, it is believed that the rare earth metals potentiate the antimicrobial activity of the anionic and zwitterionic surfactants herein, rendering them bactericidal at low concentrations to both Gram-positive and Gram-negative bacteria. As will be demonstrated more fully hereinafter, these rare earth metal/surfactant compounds provide superior, broad-scale antimicrobial agents suitable for use in a wide variety of products.

The antimicrobial compounds of the present invention comprise:

1) a rare earth metal component $M^{3+}$, and

2) a water-soluble surfactant component $RCO_2^-$.

## The Rare Earth Metal Component

The rare earth metals useful in the compounds and compositions of the instant invention have a valence state of +3 and are selected from the group consisting of scandium,

yttrium, lanthanum, cerium, praseodymium, neodymium, promethium, samarium, europium, gadolinium, terbium, dysprosium, holmium, erbium, thulium, ytterbium and lutetium. Mixtures of these metals (e.g., the so-called "didymium" salts) can also be used. Preferably, the rare earth metal is selected from lanthanum, cerium, praseodymium, neodymium, erbium, ytterbium, and mixtures thereof. Most preferably, the rare earth metal is lanthanum. Lanthanum is readily available, inexpensive and stable over the broad pH range disclosed herein.

It is noteworthy that rare earth metals having a valence state of +4, for example cerium IV, or a valence state of +2 are not effective for use in the present invention. Other polyvalent metals, such as aluminum, calcium, magnesium, iron, and indium are also ineffective as components in the instant antimicrobial compositions.

The rare earth metals herein are used in the form of their water-soluble salts, such as the chlorides, nitrates, or sulfates. The chloride salts are especially preferred.

## The Surfactant Component

The surfactants useful in the present invention are certain anionic and zwitterionic surfactants having a hydrophilic, water-solubilizing carboxylate end-group and a hydrophobic hydrocarbyl "tail".

In the general formula for the antimicrobial compounds herein, the water-soluble surfactant component is represented by the formula $RCO_2^-$, wherein R is a $C_8$-$C_{18}$ hydrocarboy ethoxylate group containing from about 1 to about 15 ethylene oxide units, a $C_8$-$C_{18}$ hydrocarbyl glyceryl ether group, a $C_8$-$C_{18}$ hydrocarbyl monoglyceride group, a $C_8$-$C_{18}$ hydrocarbyl ester group, or an ammonium phosphonium or sulfonium group of the formula

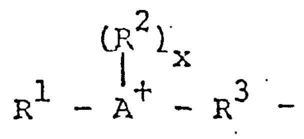 or 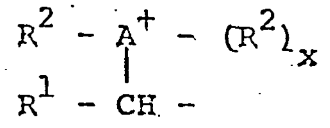 ,

wherein $R^1$ is a $C_8$-$C_{22}$ hydrocarbyl group, each $R^2$ is a $C_1$-$C_4$ hydrocarbyl group or an ethylene oxide group containing from 1 to about 5 ethylene oxide units, x is 2 when A is N or P and x is 1 when A is S, and $R^3$ is a $C_1$-$C_{10}$ hydrocarbyl group or an ethylene oxide group containing from 1 to about 10 ethylene oxide units.

As used herein, the term "hydrocarbyl" is intended to describe any straight or branched chain alkyl, alkenyl, alkynyl, alkaryl (e.g., alkyl phenyl or alkyl benzyl), or substituted hydrocarbyl (e.g., hydroxyalkyl) group. Of course, the $R^3$ substituent herein could be any of the corresponding groups based on the alkene series. It will also be recognized that for chemical stability or ease of synthesis, various short-chain alkylene (e.g., methylene or ethylene), hydroxyalkylene or alkenylene groups can be used to connect the above R substituents with the $CO_2^-$ group.

With respect to the anionic surfactant components described above, R is preferably a $C_8$-$C_{18}$ alkyl or alkyl phenyl ethoxylate group containing from 1 to about 15 ethylene oxide units, a $C_8$-$C_{14}$ alkyl glyceryl ether group, a $C_8$-$C_{14}$ alkyl monoglyceride group, or a $C_8$-$C_{14}$ alkyl ester group. More preferably R is a $C_8$-$C_{18}$ alkyl ethoxylate group containing from about 3 to about 10 ethylene oxide units. Most preferably, R is a $C_{12}$-$C_{16}$ alkyl ethoxylate group containing from about 6 to about 8 ethylene oxide units.

It is to be understood that simple alkyl and alkyl aryl carboxylates are not useful as the surfactant component herein because they form poorly soluble precipitates with the rare earth metals.

Typical examples of anionic surfactants useful herein include: the alkyl glyceryl ether carboxylates, especially those ethers of the higher alcohols derived from tallow and coconut oil; coconut oil fatty acid monoglyceride carboxylates; carboxylic acid derivatives of the reaction product of one mole of a higher fatty alcohol (e.g., tallow or coconut alcohols) and from about 3 to about 10, preferably about 6 to about 8, moles of ethylene oxide; $C_9$ alkyl phenyl ethylene oxide carboxylates containing about 4 units of ethylene oxide per molecule; and monococonutalkyl esters of succinic acid, maleic acid, and tartaric acid.

Zwitterionic surfactants useful herein, along with methods of synthesis, are disclosed in U.S. Patent 3,862,058, Nirschl, et al., issued January 21, 1975; U.S. Patent 4,051,234, Gieske, et al., issued September 27, 1977; and U.S. Patent 2,199,397, Engelmann, issued May 7, 1940, all incorporated herein by reference. Additionally, a preferred process for preparing alpha-halo carboxylic acids, from which the alpha-substituted zwitterionic surfactants herein can be derived, is disclosed in U.S. Patent 4,148,811, Crawford, issued April 10, 1979, incorporated herein by reference.

In the general formula for the zwitterionic surfactants herein, R is preferably an ammonium group. More preferably R is a $C_{12}$-$C_{22}$ alkyl dimethyl ammonio group in which $R^3$ is a $C_1$-$C_6$ alkylene group. Most preferably, R is a $C_{12}$-$C_{16}$ alkyl dimethyl ammonio group in which $R^3$ is a $C_3$-$C_5$ alkylene group.

Specific examples of other anionic and zwitterionic surfactants useful herein include: $C_{12-13}$ alkyl triethoxy methylene carboxylate; $C_9$ alkyl phenyl hexaethoxy methylene carboxylate; $C_{16}$ pentaethoxy methylene carboxylate; coconutalkyl ethoxy (avg. 7) methylene carboxylate; coconut monoglyceride methylene carboxylate; 5-(S-methyl S-dodecylsulfonio)-3-hydroxypentane-1-carboxylate; 3-(N,N-dimethyl-

BAD ORIGINAL

N-decylammonio)propane-1-carboxylate; 3-(N,N-dimethyl-N-hexadecylammonio)-2-hydroxypropane-1-carboxylate; 5-[N,N-di(3-hydroxypropyl)-n-decylammonio]2-hydroxypentane-1-carboxylate; and 4-(N,N-dimethyldodecylammonio)-butanoate.

The rare earth/surfactant compounds herein exhibit their antimicrobial properties in aqueous systems. The preparation of these compounds involves simply admixing the anionic surfactant (preferably in the form of an inorganic salt, such as the sodium, potassium or ammonium salt) or the zwitterionic surfactant, water, and the water-soluble rare earth metal salt, e.g., rare earth metal chloride (preferred), nitrate or sulfate. (In solution, the chloride, sulfate or nitrate radical would become the counter-ion for the cationic charge center of the zwitterionic surfactant.) Preferably the molar ratio of the surfactant to the rare earth metal salt is about 3:1, but ratios of from about 1:1 to about 10:1 (surfactant:rare earth metal salt) can optionally be used. Once prepared, the rare earth/surfactant compounds herein need not be isolated from solution in order to provide their desirable antimicrobial benefits, but can be used *in situ*. Since the compounds herein or their rare earth metal components precipitate from solution at pH's above about 8 (see Moeller and Kremers, *Chem. Rev.* *37*:114 (1945)), it will be appreciated that the antimicrobial compositions herein should be manufactured and used in solution at a pH of less than about 8, and preferably from about 4 to about 8. Of course, compositions intended for oral use should have a pH above about 4 to avoid damage to dental enamel. Such compositions preferably have a pH of from about 4.5 to about 8. With certain species of microorganisms, especially with *Pityrosporum ovale* which is the causative microorganism for dandruff, the compositions herein are optimally active in the pH range of 4.0-4.5.

The antimicrobials provided by the present invention can be used in a wide variety of products. Specific, but

non-limiting examples of compositions employing the instant antimicrobial agents include: oral products, such as dentifrices (toothpastes, toothpowders), mouthwashes, oral lozenges, and chewing gum, shampoos, laundry or dishwashing detergent products, hard surface cleansers and sanitizers, skin sanitizers (e.g., surgical scrubs, bar soaps), deodorants, eye drops, nose drops, and various other medicaments for use in treating human and lower animals, and the like.

Oral products prepared according to this invention comprise (a) a safe and effective amount of the aforedescribed antimicrobial; and (b) an orally-acceptable carrier. For example, the carrier for mouthwashes can be water or an organic solvent such as various alcohols, or mixtures thereof. Mouthwashes generally comprise a water/ethyl alcohol solution and optionally contain other ingredients such as standard flavors, sweeteners, humectants, and the like, as disclosed in U.S. Patent 4,067,962, Juneja, issued January 10, 1978, incorporated herein by reference. The alcohol helps solubilize the rare earth/surfactant antimicrobial, and controls product sudsing, etc.

Mouthwashes according to this invention contain about 0.01% to 5.0% of the rare earth/surfactant antimicrobial; 0% to 40% ethyl alcohol; 0% to 20% (preferably 5% to 20%) glycerine or other humectant; 0% to 2% (preferably 0.1% to 2%) sudsing agent; 0% to 0.5% (preferably 0.05% to 0.5%) sweetening agent such as saccharin; and 0% to 0.3% (preferably 0.05% to 0.3%) flavoring agent; and the balance, water.

Dentifrices prepared according to the instant invention contain the instant antimicrobial, as well as various abrasive polishing materials, sudsing agents, flavoring and sweetening agents, humectants, binders and water. See U.S. Patent 4,067,962 for disclosures of typical materials and methods for preparing dentifrices.

Certain ingredients used in many commercial dentifrice compositions are preferably avoided in dentifrices containing the present rare earth/surfactant antimicrobials. For example, soluble fluorides react with the rare earths and precipitate as the metal fluorides. Certain phosphate dentifrice abrasives are somewhat soluble and can undesirably cause precipitation of rare earth phosphates.

Various inert dentifrice abrasives useful herein are known, including, by way of example, the condensation products of urea and formaldehyde as disclosed by Cooley, et al. in U.S. Patent 3,070,510, issued December 25, 1962; silica xerogels such as those disclosed in U.S. Patent 3,538,230 to Pader, et al., issued November 3, 1970; and mineral abrasives coated with cationic polymers such as those disclosed in U.S. Patent 4,110,083, issued August 29, 1978, and in U.S. Patent 4,157,387, issued June 5, 1979, both to Benedict. The disclosures in the above patents are incorporated herein by reference.

The antimicrobial compounds herein can also be used in oral products intended for ingestion by humans and lower animals. Such products would be effective in controlling bacteria in the gastrointestinal tract. These compositions contain a safe and antimicrobially-effective amount of the compounds herein and a non-emetic carrier material. Suitable carrier materials are any of the usual pharmaceutical excipients, including water, alcohol, sugars, starches, cellulose and its derivatives, malt, gelatins and the like.

Within the realm of sound medical judgment, the dosage of the antimicrobial compound to be administered in such oral products will vary with the particular condition being treated, the severity of the condition, and the duration of treatment employed. However, single dosages can range from 1 to 100 milligrams (mg) per kg of body weight. The carrier employed in conjunction with the

antimicrobial compound is used at a concentration sufficient to provide a practical size to dosage relationship. Preferably, the pharmaceutical carrier comprises from about 0.1% to about 99% by weight of total oral composition.

The antimicrobial compositions herein are also useful in a wide variety of situations where sanitization of environmental or body surfaces is desirable. Skin sanitizers comprising safe and antimicrobially-effective amounts of the rare earth/surfactant compositions and a cosmetically-acceptable cleansing-type carrier at a pH within the range from above about 4.5 to about pH 7, and shampoo compositions comprising a safe and antimicrobially-effective amount of said compositions in an acceptable shampoo-type carrier having a pH within the range of about 4 to about 4.5, are typical of the uses of the present compositions. Likewise, hard surface sanitizing cleansers comprising the present compositions and hard surface cleanser-type carriers are another use. Of course, any of these types of products are equally appropriate for both human and veterinary use, and any of the products exemplified can be used with pets and domestic animals of all types.

As can be seen from the foregoing discussion and the following examples, the present invention provides an excellent method for controlling microorganisms, including bacteria and environmental or pathogenic fungi. The antimicrobial compounds herein are especially useful in water-containing systems at a pH in the range of 4 to about 8. Aqueous compositions containing the rare earth/surfactant compositions of this invention safely provide broad spectrum antimicrobials at usage levels in the range of ca. 8 ppm, and above, preferably ca. 25-30 ppm, and above, depending on the microorganism.

The following non-limiting examples illustrate the compositions and methods of the present invention.

All "percentages" and "parts" herein are by weight, unless otherwise specified.

Example I

A mouthwash in accordance with the present invention is formulated as follows:

| Component | Parts by Weight |
|---|---|
| Ethyl alcohol (95% in water) | 12.00 |
| Polyoxyethylene (20) sorbitan monooleate | 0.12 |
| Sodium hydroxide (10% in water) | to pH 6.5-7.0 |
| Sodium saccharin | 0.055 |
| Flavoring | 0.16 |
| La $C_{14}$ alkyl dimethyl ammoniohexanoate | 0.20 |
| Color | 0.05 |
| Sorbitol (70% in water) | 12.00 |
| Distilled water | balance to 100 |

The lanthanum $C_{14}$ alkyl dimethyl ammoniohexanoate is prepared as follows. $C_{14}$ alkyl dimethyl ammoniohexanoate is mixed with water and heated to about 40°C, with stirring, until a clear solution is obtained. Separately, a stoichiometric amount of lanthanum chloride heptahydrate is mixed with water at room temperature and stirred until a clear solution is formed. The solution of lanthanum chloride is slowly added to the first solution and stirred for about 5 minutes to form an aqueous solution of the desired compound.

When used in the normal manner to rinse the mouth, the product of Example I is effective in destroying microorganisms in the mouth and retarding the formation of dental plaque on teeth.

## Example II

A toothpaste is prepared according to the following formula:

| Component | Parts by Weight |
|---|---|
| Sorbitol (70% soln.) | 20.00 |
| Sodium saccharin | 0.21 |
| Veegum (colloidal magnesium aluminum silicate) | 0.40 |
| Precipitated urea/formaldehyde condensate (abrasive) | 30.00 |
| Flavor | 1.00 |
| Locust bean gum | 1.30 |
| Glycerine | 10.00 |
| La $C_{14}$ alkyl etheneoxy (7 avg.) carboxylate | 0.70 |
| Sodium lauryl sulfate | 1.50 |
| Distilled water | balance to 100 |

When used in the normal manner, the above toothpaste composition is effective in retarding the formation of dental plaque and produces an appreciably lower level of stain on the teeth than does the antiplaque agent chlorhexidine.

## Example III

A chewing gum in accordance with the present invention is formulated as follows:

| Component | | Parts by Weight |
|---|---|---|
| Gum Base | | 21.30 |
| Ester Gum | 6.40 | |
| Coumarone resin | 9.60 | |
| Dry latex rubber | 3.20 | |
| Paraffin wax (M.P. 180°F) | 2.10 | |
| Sugar | | 58.45 |
| Corn Syrup (Baume 45) | | 18.20 |
| Flavoring | | 1.05 |
| La $C_{16}$ alkyl dimethyl ammonioacetate | | 1.00 |

Chewing this gum in the normal manner retards the formation of dental plaque on teeth.

Example IV

A shampoo composition is prepared by blending the following components:

| Component | Parts by Weight |
| --- | --- |
| Sodium coconut alkyl glyceryl sulfonate | 15.0 |
| Sodium stearate | 8.7 |
| Perfume | 1.0 |
| La $C_{20}$ alkyl dimethyl ammoniohexanoate | 3.0 |
| Citric acid | to pH 4.0-4.2 |
| Water | to 100 |

The above product is effective in retarding and/or controlling the microorganism *Pityrosporum ovale* responsible for dandruff on the scalp.

Example V

A sanitizing, skin moisturizing composition is prepared as follows:

| Component | Parts by Weight |
| --- | --- |
| Amerlate WFA[a] | 0.75 |
| Amerlate W[b] | 1.00 |
| Stearic acid | 1.00 |
| Cetyl alcohol | 1.75 |
| Stearyl alcohol | 0.75 |
| "OH Lan"[c] | 0.75 |
| Arlacel 165[d] | 0.75 |
| Arlamol E[e] | 1.25 |

| | |
|---|---|
| 2-pyrrolidinone | 8.00 |
| Distilled water | 72.55 |
| Natrosol HH[f] | 1.00 |
| Propylene glycol | 2.50 |
| Sorbitol (70% solution in $H_2O$) | 2.50 |
| Triethanolamine | 0.65 |
| La $C_{12}$ alkyl dimethyl ammonioacetate | 2.00 |
| Titanium dioxide | 0.20 |
| Perfume and minors | to 100 |

[a]Mixture of lanolin fatty acids (Amerchol Unit of CPC International).

[b]Isopropyl ester of lanolin fatty acids (Amerchol Unit of CPC International).

[c]Hydroxylated lanolin (Amerchol Unit of CPC International).

[d]Glycerol monostearate (ICI United States, Inc.).

[e]Polyoxypropylene (15) stearyl ether (ICI United States, Inc.).

[f]Hydroxyethylcellulose (Hercules, Inc.)

When used in the normal manner, the above product remains free from microorganisms which tend to develop in many dermatological compositions.

## Example VI

A hard surface cleaning composition is prepared as follows:

| Component | Parts by Weight |
|---|---|
| 3-[N,N-dimethyl-N-coconutalkylammonio]-2-hydroxypropane-1-sulfonate | 2.0 |
| Ethoxylated (7 moles) primary coconut alcohol | 4.0 |
| Ethoxylated (5 moles) $C_{11}$-$C_{15}$ alkyl straight chain random secondary alcohol | 1.5 |

| | |
|---|---|
| Potassium cumene sulfonate | 4.0 |
| Potassium toluene sulfonate | 4.0 |
| La $C_{10}$ alkyl etheneoxy (5 avg.) carboxylate | 4.0 |
| Water | to 100 |

When used in the normal manner to scrub floors, walls, windows, doors, and the like, the above product is effective in killing microorganisms on the hard surfaces treated therewith.

Example VII

A capsule useful in controlling bacteria in the gastrointestinal tract of humans and lower animals when administered at a level of 50 mg per kg of body weight contains a 2.5 g. lanthanum $C_{14}$ alkyl dimethyl ammoniohexanoate and 1.0 g. glucose.

Substantially similar antimicrobial activity is obtained when, in Examples I through VII, the rare earth/surfactant compound is replaced with any of the following: lanthanum $C_{12}$, $C_{14}$, $C_{16}$, $C_{18}$, $C_{20}$, or $C_{22}$ dimethyl ammoniohexanoates or acetates, or with lanthanum $C_8$ $C_{10}$, $C_{12}$, $C_{14}$, $C_{16}$, or $C_{18}$ alkyl etheneoxy carboxylates having 3, 5, 7, 9, or 12 ethylene oxide units.

Comparable results are also obtained when the rare earth/surfactant compound in any of Examples I through VII is replaced with lanthanum $C_{10}$ or $C_{14}$ alkyl glyceryl ether methylene carboxylate, lanthanum $C_{12}$ alkyl monoglyceride methylene carboxylate, or lanthanum $C_{12}$ alkyl succinate.

Comparable results are also obtained when, in any of the above compositions, the lanthanum is replaced with cerium, praseodymium, neodymium, erbium, ytterbium, or mixtures thereof.

CLAIMS

1. A water-soluble rare earth metal carboxylate having the general formula I

$$(RCO_2^-)_3M^{3+} \qquad I$$

wherein M is a rare earth metal cation and R is a $C_8$-$C_{18}$ hydrocarbyl ethoxylate group containing from 1 to 15 ethylene oxide units, a $C_8$-$C_{18}$ hydrocarbyl glyceryl ether group, a $C_8$-$C_{18}$ hydrocarbyl monoglyceride group, a $C_8$-$C_{18}$ hydrocarbyl ester group, or an ammonium phosphonium or sulfonium group of the formula

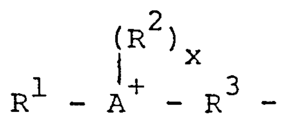

or

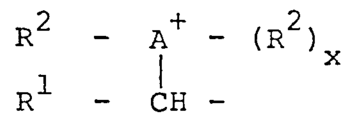

wherein $R^1$ is a $C_8$-$C_{22}$ hydrocarbyl group, each $R^2$ is a $C_1$-$C_4$ hydrocarbyl group or an ethylene oxide group containing from 1 to 5 ethylene oxide units, x is 2 when A is N or P and x is 1 when A is S, and $R^3$ is a $C_1$-$C_{10}$ hydrocarbyl group or an ethylene oxide group containing from 1 to 10 ethylene oxide units.

2. A compound according to Claim 1 characterised in that R is a $C_8$-$C_{18}$, preferably $C_{12}$-$C_{16}$, alkyl ethoxylate group containing from 3 to 10, preferably from 6 to 8 ethylene oxide units, a $C_8$-$C_{14}$ alkyl glyceryl ether group, a $C_8$-$C_{14}$ alkyl monoglyceride group, a $C_8$-$C_{14}$ alkyl ester group, or a $C_{12}$-$C_{22}$, preferably $C_{12}$-$C_{16}$ alkyl dimethyl ammonio group in which $R^3$ is a $C_1$-$C_6$, preferably $C_3$-$C_5$ alkylene group.

3. A compound according to Claim 1 or 2 characterised in that M is selected from lanthanum, cerium, praseodymium, neodymium, erbium, ytterbium, and mixtures thereof.

4. A compound according to Claim 1 characterised in that M is lanthanum and R is a $C_{12}$-$C_{16}$ alkyl ethoxylate group containing from about 6 to about 8 ethylene oxide units or R is a $C_{12}$-$C_{16}$ alkyl dimethyl ammonio group in which $R^3$ is a $C_3$-$C_5$ alkylene group.

5. A method for controlling microorganisms characterized by contacting said microorganisms with a rare earth metal carboxylate according to any of Claims 1 to 6.

6. A method according to Claim 5 characterised by being carried out at a pH of from 4 to 8.

7. An oral composition characterised by:

(a) a water-soluble rare-earth metal carboxylate according to any of Claims 1 to 4 as antimicrobial agent; and

(b) an orally-acceptable carrier.

8. A composition according to Claim 7 in the form of a toothpaste composition comprising an abrasive material, a mouthwash composition comprising water, an organic solvent, or mixtures thereof, or an oral composition intended for ingestion comprising a non-emetic ingestible carrier.

9. A cleansing composition for use as shampoo, skin sanitizer, hard surface cleaner and the like, characterised by

(a) a water-soluble rare earth metal carboxylate according to any of Claims 1 to 4 as antimicrobial agent; and

(b) a cleanser-type carrier.

10. A composition according to Claim 9 in the form of a shampoo having a pH of from 4 to 4.5 or skin sanitizer having a pH of 4.5 to 7.

European Patent Office

# EUROPEAN SEARCH REPORT

Application number
EP 81 30 1219.2

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| P | EP - A1 - 0 018 019 (PROCTER & GAMBLE)<br>* claims 1, 7 to 9 *<br>-- | 1,3,6,<br>7 | C 11 D 3/48<br>C 11 D 1/88<br>A 61 K 7/16<br>A 61 K 31/09<br>C 07 C 51/41<br>A 01 N 33/00<br>B 01 F 17/00 |
| D | GB - A - 1 475 971 (WELLA AG)<br>* claims 1, 2, 5, 6 *<br>-- | 1,3,10 | |
| D | US - A - 4 051 234 (PROCTER & GAMBLE)<br>-- | | |
| D | US - A - 4 067 962 (PROCTER & GAMBLE)<br>-- | | TECHNICAL FIELDS SEARCHED (Int. Cl.³) |
| A | GB - A - 2 001 849 (IMPERIAL CHEMICAL INDUSTRIES LTD.)<br>& DE - A1 - 2 827 666<br>-- | | A 01 N 33/00<br>A 61 K 7/00<br>A 61 K 31/00<br>B 01 F 17/00<br>C 07 C 51/00<br>C 11 D 1/00<br>C 11 D 3/00 |
| P,A | US - A - 4 217 343 (COLGATE PALMOLIVE)<br>-- | | |
| P,A | Chemical Abstracts vol. 93, no. 14, 6 October 1980<br>Columbus, Ohio, USA<br>E. BRUCHER et al. "Aminopolycarboxylates of rare earths. V. Kinetics of exchange between cerium(III) and terbium (III) ethylenediaminetetraacetate complexes and the diethylenetriaminepentaacetate ligand"<br>page 382, right column, abstract no. 138517a<br>& J. Inorg. Nucl. Chem. vol. 42, no. 5, 1980, pages 749 to 756<br>-- ./.. | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| Berlin | 13-05-1981 | SCHULTZE |

European Patent Office

# EUROPEAN SEARCH REPORT

Application number
EP 81 30 1219.2
- page 2 -

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| P,A | Chemical Abstracts vol. 93, no. 22, 1 December 1980<br>Columbus, Ohio, USA<br>Y. SAKHAROVA et al. "Study of thermal stability of urea coordination compounds of rare earth element carboxylates"<br>page 698, right column, abstract no. 214702z<br>& Term. Anal., Tezisy Dokl. Vses. Soveshch., 7th, no. 1, 1979<br>pages 63 to 65<br>--- | | TECHNICAL FIELDS SEARCHED (Int. Cl.³) |
| A | FR - A - 1 604 432 (MALMSTROM CHEMICAL CORP.) | | |